# EUROPEAN PATENT APPLICATION

(11) **EP 0 029 657 A2**
(43) Date of publication of application: **03.06.1981**
(21) Application number: 80303728.2
(22) Date of filing: 22.10.1980
(51) Int. Cl.: C07D 257/08, C07C 109/04, C07C 109/10, C07C 109/16, C07D 285/12, A01N 43/64

(54) **Pesticidal tetrazines, their use and compositions, processes for their preparation and preparation intermediates**

(30) Priority: 16.11.1979 GB 7939663; 30.09.1980 GB 8031510
(71) Applicant: FBC LIMITED, Cambridge CB2 5HU (GB)
(72) Inventor: Parsons, John Henry, Saffron Walden Essex (GB); West, Peter John, Great Shelford Cambridge (GB)
(74) Representative: Richer, David Leonard

(57) **Abstract**

The tetrazines and dihydrotetrazines of the formula: 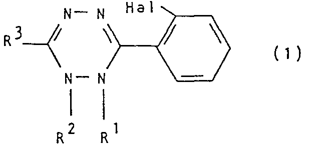 oroftheformula: 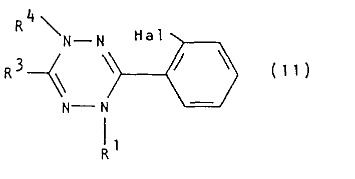 wherein R¹, R² and R', which may be the same or different, each represent hydrogen, phenyl, alkyl of 1 to 6 carbon atoms, or alkenyl or alkynyl of 3 to 6 carbon atoms, each of which may be unsubstituted or substituted by one or more halogen atoms, hydroxy groups, cyano groups, carboxy groups, alkoxycarbonyl groups of 2 to 5 carbon atoms or alkoxy groups of 1 to 4 carbon atoms; or R' and R' together represent a single bond; Hal represents fluorine, chlorine, bromine or iodine; and R³ represents 2-iodophenyl, 2,5-dihalophenyl, 2-methylthiophenyl, cyclohexyl, cyclohexylalkyl, cyclohexenyl, cyclohexadienyl, 1-naphthyl, 2-naphthyl or biphenylyl, each of the cyclohexyl, cyclohexenyl, cyclohexadienyl, naphthyl or biphenylyl moieties of which may be unsubstituted to substituted by one or more alkyl, alkoxy or alkylthio groups of 1 to 6 carbon atoms, hydroxy, mercapto or cyano groups, halogen atoms, alkanoyloxy groups of 2 to 6 carbon atoms, amino or phenylamino groups, or alkylamino or dialkylamino groups, each alkyl moiety of which is of 1 to 6 carbon atoms, are effective to combat pests, particularly acarids and their eggs.

The compounds are new and processes for their preparation are described.

## Description

This invention concerns certain tetrazines and dihydrotetrazines which are pesticidally active, particularly against acarids, their eggs and larvae, processes for their preparation, and compositions containing them.

In one aspect, this invention provides the tetrazines and dihydrotetrazines of the formula: 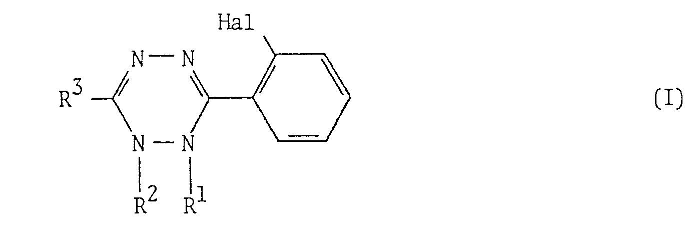 or of the formula: 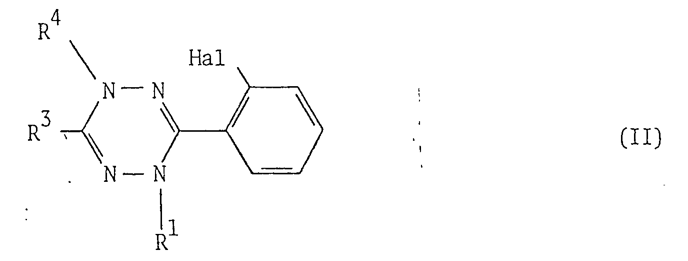 wherein _{R}^{l}, _{R}², and R⁴, which may be the same or different, each represent hydrogen, phenyl, alkyl of 1 to 6 carbon atoms, or alkenyl or alkynyl of 3 to 6 carbon atoms, each of which may be unsubstituted or substituted by one or more halogen atoms, hydroxy groups, cyano groupsr carboxy groups, alkoxycarbonyl groups of 2 to 5 carbon atoms or alkoxy groups of 1 to 4 carbon atoms; or _{R}¹ and _{R}² together represent a single bond; Hal represents fluorine, chlorine, bromine or iodine; and _{R}³ represents 2-iodophenyl, 2,5-dihalophenyl, 2-methylthiophenyl, cyclohexyl, cyclohexylalkyl, cyclohexenyl, cyclohexadienyl, 1-naphthyl, 2-naphthyl or biphenylyl, each of the cyclohexyl, cyclohexenyl, cyclohexadienyl, naphthyl or biphenylyl moieties of which may be unsubstituted or substituted by one or more alkyl, alkoxy or alkylthio groups of 1 to 6 carbon atoms, hydroxy, mercapto or cyano groups, halogen atoms, alkanoyloxy groups of 2 to 6 carbon atoms, amino or phenylamino groups, or alkylamino or dialkylamino groups, each alkyl moiety of which is of 1 to 6 carbon atoms.

In another aspect, this invention provides a method of combating pests, especially acarids, their eggs or their larvae, which comprises applying to a site either infested or liable to infestation with them an effective amount of one or more substituted tetrazines or dihydrotetrazines of formula I and/or formula II.

In a further aspect, this invention provides a pesticidal composition which comprises one or more compounds of formula I or formula II as defined hereinbefore in association with a suitable carrier and/or surface active agent.
R , R and R independently preferably represent hydrogen or alkyl of 1 to 6, especially 1 to 4, carbon atoms, which is preferably unsubstituted, for example methyl (especially preferred), ethyl, n-propyl, isopropyl or t-butyl, or _{R}¹ and R² together represent a single bond.
When R¹ or R² represents hydrogen, the compounds of formula I are of course tautomeric with compounds of formula II.
When _{R}¹, _{R}² or _{R}⁴ represents a substituted alkyl group, it may represent, for example, 2-chloroethyl, chloromethyl, cyanomethyl, carboxymethyl, methoxycarbonylmethyl or 2-methoxyethyl.

Preferred alkenyl and alkynyl groups which _{R}¹, _{R}² and/or _{R}⁴ may represent are allyl and propargyl, which are preferably unsubstituted.
When _{R}¹, _{R}² and/or _{R}⁴ represents phenyl, it is preferably unsubstituted.
Hal preferably represents bromine or, especially, chlorine.
When _{R}³ represents 2,5-dihalophenyl, both halogen atoms are preferably the same, e.g. 2,5-dichlorophenyl or 2,5-dibromophenyl.
When R³ represents cyclohexyl, it is preferably unsubstituted. When substituted, however, it is preferably substituted by one or more alkyl groups of 1 to 4, carbon atoms, e.g. methyl, ethyl, n-propyl, isopropyl or t-butyl groups. Specific mention may be made of the groups l-methylcyclohexyl, 2-methylcyclohexyl, 3-methylcyclohexyl and 4-methylcyclohexyl.
When _{R}³ represents cyclohexylalkyl, the alkyl moiety is preferably of 1 to 4, carbon atoms, especially of 1 or 2, carbon atoms. Particularly preferred such groups are thus cyclohexylmethyl and cyclohexylethyl.

The cyclohexylalkyl group is preferably unsubstituted. When substituted, however, it is preferably substituted by one or more alkyl groups of 1 to 4, carbon atoms, e.g. methyl, ethyl, n-propyl, isopropyl or t-butyl groups. Examples of such groups include 2-, 3- and 4-methylcyclohexylmethyl.
When _{R}³ represents cyclohexenyl, it may be a 1-cyclohexen-1-yl, a 2-cyclohexen-1-yl, or especially a 3-cyclohexen-l-yl group. It is preferably unsubstituted. When substituted, however, it is preferably substituted by one or more alkyl groups of 1 to 4, carbon atoms, e.g. methyl, ethyl, n-propyl, isopropyl or t-butyl groups. Examples of such groups include 3-methyl-3-cyclohexen-1-yl and 4-methyl-3-cyclohexen-1-yl.
When R³ represents cyclohexadienyl, it may be any appropriate such group. Specifically preferred are 2,5-cyclohexadien-1-yl and 1,4-cyclohexadien-1-yl groups. The group is preferably unsubstituted. When substituted, however, it is preferably substituted by one or more alkyl groups of 1 to 4, carbon atoms, e.g. methyl, ethyl, n-propyl, isopropyl or t-butyl groups. Examples of such groups include 2-, 3- and 4-methyl-2,5-cyclohexadien-l-yl.
When _{R}³ represents a naphthyl group, it is preferably unsubstituted, and is desirably a 1-naphthyl group.
When R³ represents a biphenylyl group, it is preferably unsubstituted, and is desirably a 2-biphenylyl group.

The compounds of formula I or formula II wherein R³ represents 2-iodophenyl, 2,5-dihalophenyl, 2-methylthiophenyl or cyclohexyl (substituted or unsubstituted) represent an especially preferred group of compounds which exhibit particularly high activity, especially when, as preferred, _{R}¹ and _{R}² together represent a bond or one of _{R}¹, _{R}² and _{R}⁴ represents methyl while the other represents hydrogen.

The compounds of formula I or II wherein R³ represents unsubstituted cyclohexyl are especially preferred, particularly when R¹ and R 2 together represent a bond or one of _{R}¹, _{R}² and R⁴ represents methyl while the other represents hydrogen, and more particularly when Hal represents bromine or, especially, chlorine. These compounds possess sterilant activity on mite adults and also possess foliar systemic activity on plants. Particular mention may be made of the specifically preferred compound 3-cyclohexyl-6-(2-chlorophenyl)-1,2,4,5-tetrazine.

In another aspect, this invention provides a process for the preparation of the compounds of formula I wherein _{R}¹ and _{R}² represent other than a single bond, in which process a substituted azine of the formula: 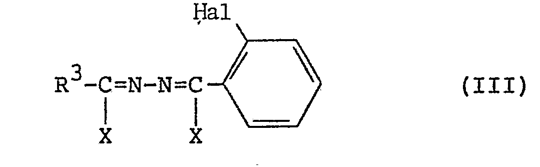
(wherein _{R}³ and Hal are as defined hereinbefore and each X represents a leaving group) is reacted with a hydrazine of the formula: 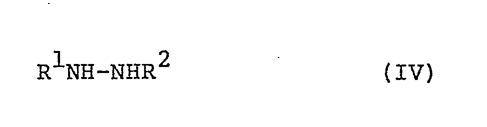
(wherein _{R}¹ and _{R}² are as defined hereinbefore but do not together represent a bond) to give the desired compound.

The reaction is conveniently carried out at an elevated temperature, e.g. from 30° to 100°C, especially 50° to 80°C, in an appropriate solvent medium, e.g. an alkanol, e.g. ethanol, an ether, e.g. tetrahydrofuran, or a hydrocarbon.

X preferably represents halogen (especially chlorine or bromine), cyano, or C 1 to 4 alkoxy (especially methoxy).

The reaction of the compounds of formulae III and IV appears to give rise (when R¹ is not the same as _{R}²) to a mixture of isomeric products. The present invention extends of course to cover the isomeric structures which may be produced.

The substituted azines of formula III employed as starting materials in the above process may themselves be prepared by a process in which a compound of the formula: 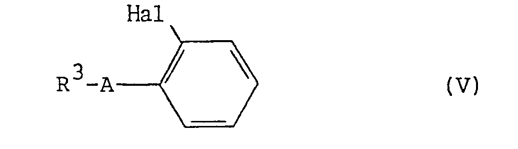 (wherein R³ and Hal are as defined hereinbefore, and A represents -CONH-NHCO-, -CONH-N=CH-, -CH=N-NHCO- or -CH=N-N=CH-) is halogenated to give the desired compound of formula III wherein X represents halogen, and the product is, if desired, reacted with an anion forming compound to give the corresponding compound where X represents a different leaving group.

When A represents -CONH-NHCO-, the halogenating agent is preferably a phosphorus pentahalide, especially phosphorus pentachloride or phosphorus pentabromide, and is preferably employed in a suitable solvent medium, for example a hydrocarbon or halogenated hydrocarbon, e.g. carbon tetrachloride or o-dichlorobenzene. The reaction is conveniently effected at elevated temperature, e.g. from 70-150°, especially from 90-110°C.

When A represents -CONH-N=CH- or -CH=N-NHCO-, the halogenation is desirably achieved in two stages by reaction of the compound of formula V with a thionyl halide, especially thionyl chloride, and with the halogen itself, especially chlorine.

When A represents -CH=N-N=_{CH}-, the halogenation is conveniently achieved in a single step reaction with the desired halogen in an appropriate solvent medium, for example acetic acid.

The compounds of formula V where A represents -CONH-NHCO- may be prepared by a process in which a hydrazide of the formula R³(or R⁶)CONHNH₂ is reacted with a compound of the formula R (or R³)COY, where _{R}³ is as defined hereinbefore, R⁶ represents 2-halophenyl, and Y represents halogen (especially chlorine or bromine), hydroxy, C 1 to 6 alkoxy or a group _{R}⁶(or _{R}³)COO- to give the desired compound.

The reaction is preferably carried out in the presence of a base, e.g. an alkali-metal hydroxide or carbonate, e.g. sodium hydroxide or carbonate, or an amine, e.g. triethylamine.

The reaction is conveniently effected at a temperature of from 5 to 50°C.

The compounds of formula V where A represents -CONH-N=CH-or -CH=_{N}-_{NHCO}- may be prepared by reaction of a hydrazide of the formula R³CONHNH₂ or R⁶CONHNH₂ with an aldehyde respectively of the formula R⁶CHO or R³CHO.

The hydrazides of formula R³(or R⁶)CONHNH₂ employed as starting materials may be prepared by conventional processes, for example by reaction of hydrazine with one molar proportion of the appropriate acid or ester _{R}³(or _{R}⁶)COO_{R} where R represents hydrogen or alkyl, especially of 1 to 4 carbon atoms.

The compounds of formula I wherein _{R}¹ and _{R}² together represent a single bond may be prepared from the corresponding compounds of formula I wherein at least one of _{R}¹ and _{R}² represents hydrogen by reaction thereof with a suitable oxidising agent.

The preferred oxidising agent when R¹ and R² both represent hydrogen is an alkali-metal nitrite, e.g. sodium nitrite, employed in the presence of an acid, and the reaction is desirably carried out in an appropriate solvent medium which is inert under the reaction conditions employed, e.g. glacial acetic acid. However, many other oxidising agents may be appropriate, particularly when one of R^{l} and _{R}² is other than hydrogen (especially alkyl, e.g. methyl), for example air, permanganates (e.g. potassium permanganate), chromium trioxide, dichromates (e.g. sodium or potassium dichromate), persulphates (e.g. sodium persulphate), periodates (e.g. sodium periodate), ceric ammonium nitrate, lead tetraacetate, hypochlorites (e.g. sodium hypochlorite), chlorine, bromine, Caro's acid, chromyl chloride, ceric sulphate, sodium bismuthate, iodates (e.g. potassium iodate) and bromates (e.g. potassium bromate).

The conditions employed will in general be those normally employed for oxidation reaction using such reagents. Thus, for example, the solvents (if any) employed should be such that they are inert with respect to the reactants under the conditions employed.

Suitable solvents for the oxidation reaction include glacial acetic acid, water, alkanols such as ethanol, and halogenated hydrocarbons, e.g. dichloromethane.

The oxidation may be conveniently effected at a temperature of from 0 to 150°C, preferably at a temperature of from 20 to 120°C, except where the oxidising agent is an alkali-metal nitrite, when the temperature employed is preferably 5-30°C, especially 10-20°C.

In another aspect, this invention provides a process for the preparation of the compounds of formula II wherein a thiadiazolium salt of the formula: 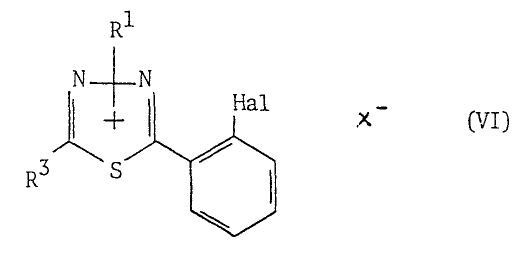 (wherein _{R}¹, R³ and Hal are as defined hereinbefore) and X represents an anion, is reacted with a hydrazine of formula R⁴NHNH₂ (wherein R⁴ is as defined hereinbefore) to give the desired compound.

The reaction is desirably effected in the presence of a solvent, e.g. an alkanol such as ethanol, and is preferably carried out at a temperature of from 20°C to the reflux temperature, e.g. 100°C.

X may represent any suitable anion, but is preferably halide, especially chloride, sulphate or p-toluenesulphonate.

The thiadiazolium salts of formula VI may themselves be prepared by a process in which a corresponding thiadiazole of the formula: 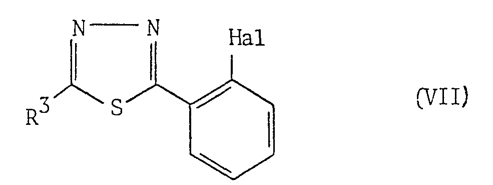 (wherein _{R}³ and Hal are as defined hereinbefore) is reacted with the appropriate compound of formula R¹X to give the desired compound.

The reaction may, if desired, be effected in an appropriate solvent medium, e.g. an aromatic hydrocarbon, e.g. toluene, or an alkanol such as ethanol, and at a temperature of from 60°C to the reflux temperature, e.g. 180°C.

The thiadiazoles of formula VII may themselves be prepared either by reaction of a compound of formula V wherein A represents -CONH-NHCO- with phosphorus pentasulphide, or by reaction of a compound of formula III with a sulphide, for example sodium sulphide or phosphorus pentasulphide, especially the former, or a hydrosulphide to give the desired compound.

The reaction involving phosphorus pentasulphide is desirably effected in an appropriate solvent medium, for example an aromatic hydrocarbon such as toluene. It may also be effected in the presence of a tertiary organic base. The base may act as the solvent medium, and may for example be pyridine, triethylamine or dimethylaniline. The reaction is desirably effected at a temperature of from 80°C to the reflux temperature, e.g. 120°C.

The sulphide or hydrosulphide employed in the reaction with the compound of formula III may alternatively be an alkali-metal sulphide or hydrosulphide, e.g. sodium sulphide or potassium hydrosulphide, and the reaction is desirably effected in an appropriate solvent medium, e.g. an alkanol, e.g. ethanol, or a polar solvent, e.g. dimethylformamide, and at a temperature of from 5°C to the reflux temperature, e.g. 80°C.

The compounds of formula I and formula II are of general use as acaricides and/or ovicides. They are of particular use against the eggs and larvae of acarids which attack plants, particularly the eggs of the red spider mite, Panonychus ulmi, but also against the eggs and larvae of other mite species, e.g. Tetranychus pacificus, Tetranychus urticae, Tetranychus cinnabarinus, Phyllocoptrata oleivora, Eutetranychus banski, Panonychus citri, Tetranychus MacDanieli, Tetranychus desertorum, Tetranychus turkestani, Tetranychus tumidus, and Eotetranychus carpini. They also exhibit, however, activity against ticks and mites on animals, for example cattle tick (Boophilus), sheep tick (Ixodes), soft tick (Rhipicephalus) and poultry mites. In addition, they exhibit activity against aphids, particularly aphis fabae (blackfly), megoura viciae (vetch aphid), sitobion avenae (grain aphid) and myzus persicae (peach potato aphid). Surprisingly, the compounds appear to be of useful low activity against predatory mites which feed on the mites which attack plants.

The compounds are normally employed in the form of compositions.

The compositions of the invention will normally be produced initially as formulations containing from 0.5 to 99%, preferably from 0.5 to 85% by weight, more usually from 10 to 50% by weight, of the active compounds, which are diluted if necessary before application to the locus to be treated such that the concentration of active ingredient in the formulation applied is from 0.05 to 5% by weight.

The substituted tetrazines of formula I and formula II are generally water insoluble and may be formulated in any of the ways commonly adopted for insoluble compounds.

For example, they may be dissolved in a water immiscible solvent, for example a high boiling hydrocarbon, as carrier, suitably containing dissolved emulsifying agents so that the composition acts as a self-emulsifiable oil on addition to water.

The substituted tetrazines may alternatively be admixed with a wetting agent with or without a solid carrier to form a wettable powder which is soluble or dispersible in water, or may be mixed with just a solid carrier to form a solid product.

An aqueous suspension concentrate may alternatively be prepared by grinding the compounds with water, a wetting agent and a suspending agent.

Solid carriers with which the substituted tetrazines may be incorporated include clays, sands, talc, mica or solid fertilizers, such products either comprising dust or larger particle size materials.

The surface active agents used may comprise anionic surface active agents, for example mono- or di-esters of phosphoric acid with fatty alcohol ethoxylates or salts of such esters, fatty alcohol sulphates such as sodium dodecyl sulphate, ethoxylated fatty alcohol sulphates, ethoxylated alkylphenyl sulphates, lignin sulphonates, petroleum sulphonates, alkylaryl sulphonates such as alkyl-benzene sulphonates or lower alkyl-naphthalene sulphonates, salts of sulphonated naphthalene-formaldehyde condensates, salts of sulphonated phenol-formaldehyde condensates, or more complex sulphonates such as the amide sulphonates, e.g. the sulphonated condensation product of oleic acid and N-methyl taurine or the dialkyl sulphosuccinates, e.g. the sodium sulphonate of dioctyl succinate.

The surface active agents may also comprise non-ionic agents, for example, condensation products of fatty acid esters, fatty alcohols, fatty acid amides or alkyl-substituted phenols with ethylene oxide, fatty esters of polyhydric alcohol ethers, e.g. sorbitan fatty acid esters, condensation products of such esters with ethylene oxide, e.g. polyoxyethylene sorbitan fatty acid esters, block copolymers of ethylene oxide and propylene oxide, acetylenic glycols such as 2,4,7,9-tetramethyl-5-decyn-4,7-diol, or ethoxylated acetylenic glycols.

The surface active agents may also comprise cationic agents, for example, alkyl- and/or aryl-substituted quarternary ammonium compounds such as cetyl trimethylammonium bromide, or ethoxylated tertiary fatty amines.

Preferred surface active agents include ethoxylated fatty alcohol sulphates, lignin sulphonates, alkyl-aryl sulphonates, salts of sulphonated naphthalene-formaldehyde condensates, salts of sulphonated phenol-formaldehyde condensates, sodium oleoyl N-methyltauride, dialkyl sulphosuccinates, alkyl phenol ethoxylates, and fatty alkyl ethoxylates.

The pesticidal composition may be in the form of an aerosol composition, suitably using a cosolvent and a wetting agent, in addition to the propellant, which is suitably a polyhalogenated alkane such as dichlorodifluoromethane.

The compositions according to the present invention may contain in addition to the substituted tetrazines other active insecticides, acaricides, ovicides, bactericides, fungicides or synergists. It has been found that particular advantages are obtained with mixtures with other acaricides, especially those which combat the motile stages, e.g. amitraz, azocyclotin, benzoximate, binapacryl, bromopropylate, calcium cyanamide, chlordimeform, chlorfenethol, chlorfenson, chlorobenzilate, chloromethi- uron, chloropropylate, cyhexatin, dicofol, dienochlor, dinobuton, fenbutatin oxide, fenson, formetanate, propargite, tetradifon, tetranactin, or tetrasul, and particularly where the compound of formula I is 3-cyclohexyl-6-(2-chlorophenyl)-1,2,4,5-tetrazine.

Synergists with which the present compounds may be admixed include piperonyl butoxide, piprotal, propyl isome and sesamin. Other pesticides with which the compounds may be admixed include the pyrethroids, e.g. pyrethrin I or II, cinerin I or II, jasmolin I or II, allethrin, barthrin, dimethrin, tetramethrin, resmethrin, biores- methrin, decamethrin, alpermethrin, permethrin, fenvaler- ate or fluvalinate, and organophosphorus compounds such as dimethoate and malathion, carbamates such as bendiocarb, carbaryl and propoxur, and chlorinated compounds such as BHC, dicofol and tetradifon. The compounds may also be admixed with the pesticides fenazaflor, formetanate, chlordimeform, chlorobenzilate, quinomethionate, endosulfan, chlorodecone, chloropropylate, bromopropylate, thioquinox, dinocap, binapacryl, dinobuton, naled, mono- crotophos, demeton, phorate, oxydemeton-methyl, ethion, formothion, ethoate-methyl, cyanthoate, EPN, carbophenothion, methidathion, chlorpyrifos, diazinon, azinphos-ethyl, azinphos-methyl, fenson, tetrasul, chlorbenside, chlorfen- sulphide, aldicarb, methomyl, methiocarb or benomyl.

The method of combating pests, particularly acarids, their eggs or their larvae, provided by the present invention may be employed at any site where infestations of such pests are present or are liable to occur. Thus, they may be applied, for example, to plants or the soil.

Plants which may be treated include food crops, for example, fruit trees and cereals, e.g. apples, pears, apricots, citrus fruits, maize, wheat or barley, beans, sugar beet, potatoes, carrots, or greenhouse crops, e.g. peppers, tomatoes, cucumbers, melons or strawberries, plantation crops, e.g. cotton or tobacco and ornamental crops.

In their various applications the compounds of formula I and formula II may be used at various rates; thus, for example, for the treatment of plants for the control of pests on plants the compounds are suitable applied at a rate of about 0.25-16 ounces per acre (17-1120g per hectare) or at a concentration of 1 to 2000 ppm as appropriate, e.g. 100 to 1000 ppm, and preferably 0.5-4 ounces per acre (35-280g per hectare). Normally the compounds will be applied to the foliage of plants, but systemic activity has also been observed when applied to the soil around the base of the plants.

The following Examples are given merely to illustrate the present invention. The temperatures given therein are in °C, and parts and percentages are by weight.

### Example 1

### 3-Cyclohexyl-6-(2-chlorophenyl)-1,2-dihydro-1(or 2)-methyl-1,2,4,5-tetrazine

### (a) N-Cyclohexylcarbonyl-N'-(2-chlorobenzoyl)hydrazine

2-Chlorobenzohydrazide (51.2g) was added to sodium hydroxide (12g) in water (250 ml), and cyclohexanecarbonyl chloride (48g) was added dropwise with stirring and cooling in ice. The stirring was continued for 1 hour at room temperature and the formed solid was filtered off, washed with warm water and dried in vacuo to give 46.6g of desired product.

### (b) 2-Cyclohexyl-5-(2-chlorophenyl)-1,3,4-thiadiazole

The product of stage (a) (46.5g) was dissolved in pyridine (230 ml), phosphorus pentasulphide (77.5g) was added portionwise with stirring, and the mixture was refluxed for 48 hours and allowed to cool. The formed solid was filtered off, washed with water and acidified with hydrochloric acid with cooling in ice. The filtrate was evaporated down, diluted with water and more solid was filtered off. The product was recrystallised from isopropyl alcohol/water to yield 21g.

### (c) 2-Cyclohexyl-5-(2-chlorophenyl)-3(or 4)-methyl-1,3,4-thiadiazolium methyl sulphate

The product of stage (b) (21g) and dimethyl sulphate (9.6g) were heated on a steam bath for 2 hours and then at 150°C for 2 hours. The melt was then cooled, triturated with ethyl acetate and extracted with acetone. The acetone was evaporated to dryness to give 21.2g of desired product.

### (d) 3-Cyclohexyl-6-(2-chlorophenyl)-1,2-dihydro-1(or 2)-methyl-1,2,4,5-tetrazine

3(or 4)-Methyl-2-cyclohexyl-5-(2-chlorophenyl)-1,3,4-thiadiazolium methyl sulphate (21.2g) was dissolved in ethanol (100 ml), hydrazine (5.5g) was added, and the mixture was refluxed for 22 hours. It was then allowed to cool, the ethanol was evaporated off, and the residue was washed with water, taken up in ethyl acetate, washed with water, dried over magnesium sulphate and evaporated down to give 12.0g of desired product, mp 130-132°C.

### Example 2

### 3-Cyclohexyl-6-(2-chlorophenyl)-1,2,4,5-tetrazine

The product of Example 1 (12g) was dissolved in acetic acid (70 ml), and the solution was added to potassium dichromate (13.4g) in acetic acid (130 ml). The mixture was then heated on a steam bath for 1 hour, allowed to cool and poured into water (750 ml). The solid was then filtered off, washed with water and recrystallised from ethanol to give 4.5g of the desired product, mp 90-91°C.

### Examples 3-17

By methods analogous to those of Examples 1 and 2, the following compounds were prepared:
3. 3-(2-Iodophenyl)-6-(2-chlorophenyl)-1(or 2)-methyl-l,2-dihydro-1,2,4,5-tetrazine, mp 153°C.
4. 3-(2-Iodophenyl)-6-(2-chlorophenyl)-1,2,4,5-tetrazine, mp 145°C.
5. 3-(2-methylthiophenyl)-6-(2-chlorophenyl)-l(or 2)-methyl-1,2-dihydro-1,2,4,5-tetrazine, mp 170°C.
6. 3-(2,5-Dichlorophenyl)-6-(2-chlorophenyl)-1,2-dihydro- l(or 2)-methyl-l,2,4,5-tetrazine, mp 166-168°C.
7. 3-(2,5-Dichlorophenyl)-6-(2-chlorophenyl)-1,2,4,5-tetrazine, mp 180-182°C.
8. 3-Cyclohexylmethyl-6-(2-chlorophenyl)-1,2,4,5-tetrazine,mp 66°C.
9. 3-Cyclohexyl-6-(2-bromophenyl)-1,2-dihydro-1(or 2)-methyl-1,2,4,5-tetrazine, mp 108°C.
10. 3-Cyclohexyl-6-(2-bromophenyl)-1,2,4,5-tetrazine, mp 89-91°C.
11. 3-Cyclohexyl-6-(2-fluorophenyl)-1,2-dihydro-l(or 2)-methyl-1,2,4,5-tetrazine, mp 94°C.
12. 3-Cyclohexyl-6-(2-fluorophenyl)-1,2,4,5-tetrazine, mp 53-56°C.
13. 3-(1-Methylcyclohexyl)-6-(2-chlorophenyl)-1,2,4,5-tetrazine, mp 69°C.
14. 3-(1-Naphthyl)-6-(2-chlorophenyl)-1,2-dihydro-1(or 2)-methyl-l,2,4,5-tetrazine, brown oil.
15. 3-(1-Naphthyl)-6-(2-chlorophenyl)-1,2,4,5-tetrazine, mp 142°C.
16. 3-(2-Biphenylyl)-6-(2-chlorophenyl)-1,2,4,5-tetrazine, mp 54-58°C.

### Example 17

### 3-Cyclohexyl-6-(2-chlorophenyl)-1,2,4,5-tetrazine (alternative route)

### (a) N-(α:2-dichlorobenzylidene)-N'-[chloro(cyclohexylmethylene)]hydrazine

N-Cyclohexanoyl-N'-(2-chlorobenzoyl)hydrazine (28g), prepared as in Example l(a) suspended in carbon tetrachloride (100 ml) was added over 10 minutes to a suspension of phosphorus pentachloride (46g) in carbon tetrachloride (100 ml). The mixture was refluxed for 10 minutes and the carbon tetrachloride and phosphoryl chloride were removed in vacuo. The residue was treated with 60/80 petrol (150 ml) and filtered. The petrol was then evaporated off in vacuo to give 29.7g of the desired product.

### (b) 3-Cyclohexyl-6-(2-chlorophenyl)-1,2-dihydro-l(or 2)-methyl-1,2,4,5-tetrazine

Methyl hydrazine (18.4g) and ethanol (200 ml) were heated to reflux. Then the heating was stopped and the product of stage (a) (29.7g) in ethanol (50 ml) was added to maintain slow reflux. The mixture was then evaporated to low bulk and cooled in ice. Ice was added to the mixture slowly to give a solid which was filtered off, washed with water, then with 15% isopropyl alcohol/water. Drying in vacuo gave 18.lg of desired product, mp 130-132⁰C.

### (c) 3-Cyclohexyl-6-(2-chlorophenyl)-1,2,4,5-tetrazine

The product of stage (b) was oxidised by the method of Example 2 to give a product identical to that of Example 2. Example 18

### 3-(3(or 4)-Methyl-3-cyclohexen-1-yl)-6-(2-chlorophenyl)-1,2,4,5-tetrazine

### (a) (3(or 4)-Methyl-3-cyclohexenecarboxylic acid hydrazide

3(or 4)-Methyl-3-cyclohexene carboxylic acid (56g) was dissolved in a mixture of n-butanol (30 ml) and toluene (20 ml). Hydrazine hydrate (20 ml) was then added, and the mixture was refluxed under a Dean and Stark apparatus until the theoretical quantity of water had been evolved. The mixture was then left to cool and crystallise to give 43.4g of the desired product.

### (b) 1-(2-Chlorobenzoyl)-2-(4-methylcyclohex-3-enoyl)-hydrazine

Sodium hydroxide (8.8g) was dissolved in water (300 ml) and the hydrazide product of stage (2) (30.8g) was added. The suspension was stirred and cooled to 5°C. Then o-chlorobenzoyl chloride (38.3g) was added dropwise. The mixture was stirred for 5 hours, the solid was filtered off and washed with water then with sodium bicarbonate solution. Drying yielded 57.2g of the desired product, mp 204-208°C.

### (c) N-(α:2-dichlorobenzylidene)-N'-[chloro(3(or 4)-methyl-3-cyclohexen-I-yl)methylene]hydrazine

The product of stage (b) (28.2g) was added portionwise to phosphorus pentachloride (42g) in carbon tetrachloride (250 ml) at reflux. The mixture was refluxed for 5 minutes and the carbon tetrachloride was evaporated off. Petrol was added to the residue and was filtered. The filtrate was evaporated to give an orange oil (30.1g) of the desired product.

### (d) 3-(3(or 4)-Methyl-3-cyclohexen-1-yl)-6-(2-chlorophenyl)-l(or 2)-methyl-1,2-dihydrotetrazine

The dichloroazine product of stage (c) (30.1g) in ethanol (100 ml) was added portionwise to methyl hydrazine (17.0g) in ethanol (400 ml) at reflux. The mixture was refluxed for 10 minutes, then the ethanol was evaporated off to low bulk. Ice was added to the residue to give an orange oil which was extracted into ether, then washed with water, dried over magnesium sulphate and evaporated to give 16.2g of the desired product as a yellow oil.

### (e) 3-(3(or 4)-Methyl-3-cyclohexen-1-yl)-6-(2-chlorophenyl)-1,2,4,5-tetrazine

The product of stage (d) (16.2g) in acetic acid (120 ml) was treated with potassium dichromate (15g). The product was chromatographed on silica gel and recrystallised from methanol to yield 0.6g of desired product, mp 98-100 C as a mixture of isomers.

### Example 19

Wettable powders were prepared with the following ingredients:

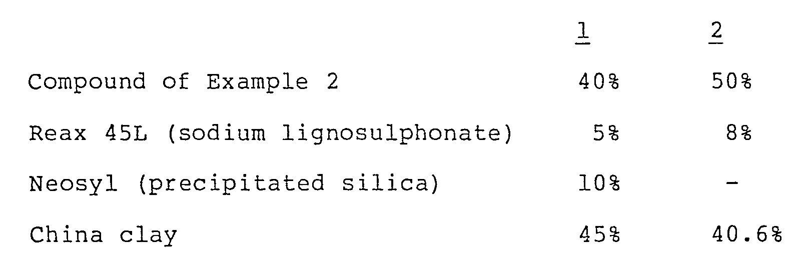

### Example 20

An emulsifiable concentrate was prepared with the following ingredients:

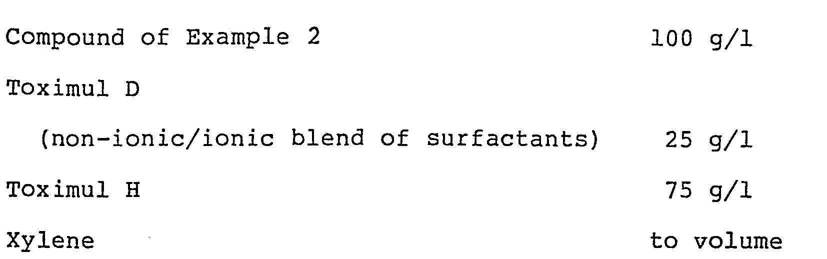

### Example 21

An aqueous suspension concentrate was prepared with the following ingredients:

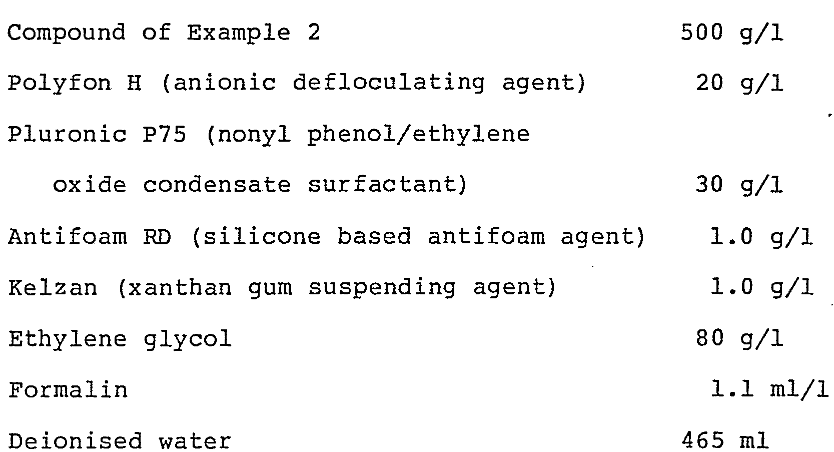

### Example.A

Aqueous acetone solutions containing 1000, 300, 100, 10, 3 and 0.3 ppm respectively of each of the compounds listed below together with 500 ppm of the wetting agent Lissapol NX (a nonylphenol/ethylene oxide condensate) were applied to 2 cm diameter discs cut from the leaves of french bean plants, Phaseolus vulgaris, each infested with 50-100 eggs of the greenhouse red spider mite, Tetanychus telarius. The treated leaf discs and mite eggs, together with controls treated with wetting agent alone were held at 25°C for 5 days on moist filter paper. The percentage mortality of the mite eggs was then recorded, and the LC₅₀ was determined and scored according to the following scale:

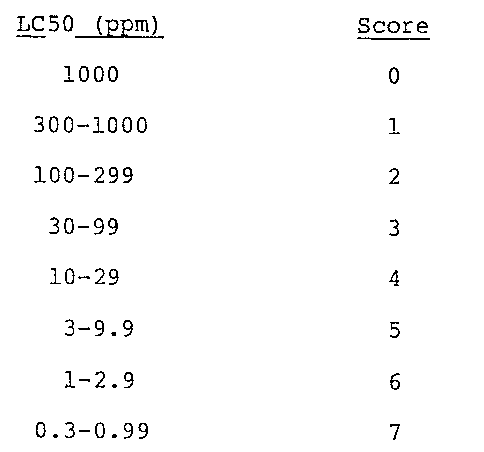

The results obtained were as follows:

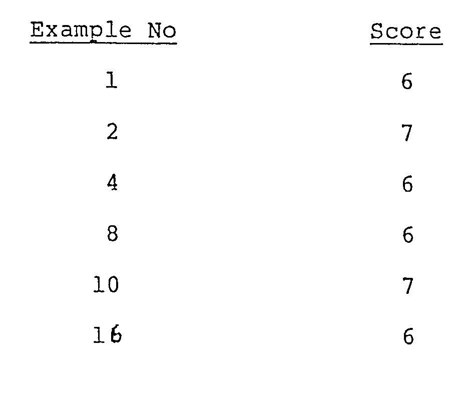

## Claims

1. The tetrazines and dihydrotetrazines of the formula: 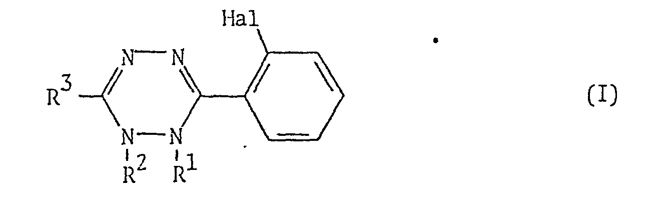 or of the formula: 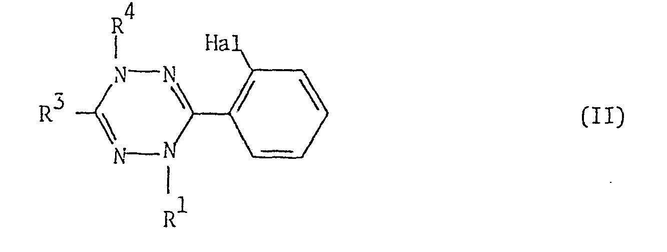 wherein _{R}^{l}, _{R}² and R⁴, which may be the same or different, each represent hydrogen, phenyl, alkyl of 1 to 6 carbon atoms, or alkenyl or alkynyl of 3 to 6 carbon atoms, each of which may be unsubstituted or substituted by one or more halogen atoms, hydroxy groups, cyano groups, carboxy groups, alkoxycarbonyl groups of 2 to 5 carbon atoms or alkoxy groups of 1 to 4 carbon atoms; or _{R}¹ and _{R}² together represent a single bond; Hal represents fluorine, chlorine, bromine or iodine; and _{R}³ represents 2-iodophenyl, 2,5-dihalophenyl, 2-methylthiophenyl, cyclohexyl, cyclohexylalkyl, cyclohexenyl, cyclohexadienyl, 1-naphthyl, 2-naphthyl or biphenylyl, each of the cyclohexyl, cyclohexenyl, cyclohexadienyl, naphthyl or biphenylyl moieties of which may be unsubstituted or substituted by one or more alkyl, alkoxy or alkylthio groups of 1 to 6 carbon atoms, hydroxy, mercapto or cyano groups, halogen atoms, alkanoyloxy groups of 2 to 6 carbon atoms, amino or phenylamino groups, or alkylamino or dialkyl amino groups, each alkyl moiety of which is of 1 to 6 carbon atoms.

2. The compounds according to claim 1 wherein _{R}¹, _{R}² and R each represent hydrogen or alkyl of 1 to 4 carbon atoms, or Rand R² together represent a bond.

3. The compounds according to claim 1 or claim 2 wherein Hal represents chlorine or bromine.

4. The compounds according to any of claims 1 to 3 wherein _{R}³ represents 2-iodophenyl, 2,5-dihalophenyl or 2-methylthiophenyl.

5. The compounds according to any of claims 1 to 3 wherein _{R}³ represents unsubstituted cyclohexyl.

6. The compounds:
3-cyclohexyl-6-(2-chlorophenyl)-1,2-dihydro-1(or 2)-methyl-l,2,4,5-tetrazine;
3-(2-iodophenyl)-6-(2-chlorophenyl)-1,2-dihydro- l(or 2)-methyl-1,2,4,5-tetrazine;
3-(2-iodophenyl)-6-(2-chlorophenyl)-1,2,4,5-tetrazine;
3-(2-methylthiophenyl)-6-(2-chlorophenyl)-1,2-dihydro- l(or 2)-methyl-l,2,4,5-tetrazine;
3-(2,5-dichlorophenyl)-6-(2-chlorophenyl)-1,2-dihydro- l(or 2)-methyl-l,2,4,5-tetrazine;
3-(2,5-dichlorophenyl)-6-(2-chlorophenyl)-1,2,4,5-tetrazine;
3-cyclohexylmethyl-6-(2-chlorophenyl)-1,2,4,5-tetrazine;
3-cyclohexyl-6-(2-bromophenyl)-1,2-dihydro-1(or 2)-methyl-l,2,4,5-tetrazine;
3-cyclohexyl-6-(2-bromophenyl)-1,2,4,5-tetrazine;
3-cyclohexyl-6-(2-fluorophenyl)-1,2-dihydro-1-(or 2)-methyl-l,2,4,5-tetrazine;
3-cyclohexyl-6-(2-fluorophenyl)-1,2,4,5-tetrazine;
3-(1-methylcyclohexyl)-6-(2-chlorophenyl)-1,2,4,5-tetrazine; and
3-(3(or 4)-methyl-3-cyclohexen-1-yl)-6-(2-chlorophenyl)-1,2,4,5-tetrazine.

7. 3-Cyclohexyl-6-(2-chlorophenyl)-1,2,4,5-tetrazine.

8. A pesticidal composition which comprises from 0.5 to 99% by weight of one or more compounds according to any of claims 1 to 7 in association with a suitable carrier and/or surface active agent.

9. A method of combating acarids, their eggs or their larvae, which comprises applying to a site either infested or liable to infestation with them, an effective amount of one or more compounds according to any of claims 1 to 7.

10. A process for the preparation of a compound as claimed in claim 1 wherein:
(a) a compound of formula I wherein R¹ and R² represent other than a single bond is produced by reaching a substituted azine of the formula: 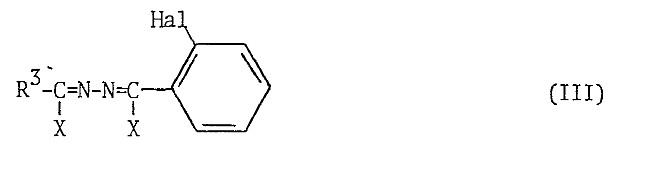 (wherein _{R}³ and Hal are as defined in claim 1 and each X represents a leaving group) is reacted with a hydrazine of the formula: 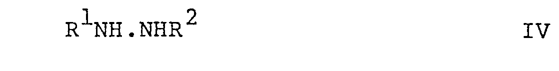 . (wherein R¹ and R² are as defined in claim 1 but do not together represent a bond); or
(b) a compound of formula I wherein R¹ and R² together represent a bond is produced by reacting a compound of formula I wherein at least one of R¹ and R² represents hydrogen with a suitable oxidising agent; or
(c) a compound of formula II is produced by reacting a thiadiazolium salt of the formula: 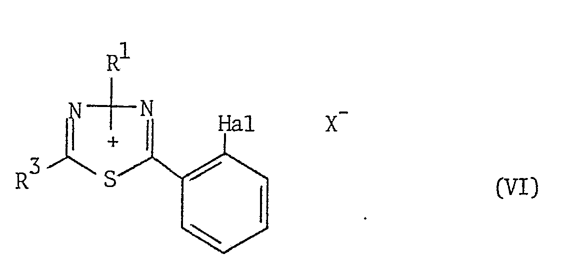 (wherein _{R}¹, _{R}³ and _{H}al are as defined in claim 1, and X⁻ represents an anion) with a hydrazine of formula R⁴NHNH₂ (wherein R⁴ is as defined in claim 1) to give the desired compound.

11. A compound of formula III, V, VI or VII as defined herein.
